# DEMANDE DE BREVET EUROPEEN

(11) **EP 1 450 158 A1**
(43) Date de publication de la demande: **25.08.2004**
(21) Numéro de dépôt: 04290458.1
(22) Date de dépôt: 20.02.2004
(51) Int. Cl.: G01N 33/46, G01N 27/04, G01N 31/22

(54) **Procédé et dispositif de mesure de l'humidité du bois**

(30) Priorité: 24.02.2003 FR 0302215
(71) Demandeur: Couvreur, Michel, 76260 EU (FR)
(72) Inventeur: Couvreur, Michel, 76260 EU (FR)
(74) Mandataire: Lhuillier, René

(57) **Abrégé**

Le dispositif (1) de mesure de l'humidité du bois, comprend des circuits de sonde (21), agencés pour être plaqués contre une surface humide d'une pièce de bois à tester afin d'en recevoir un flux d'un courant fonction d'un taux d'humidité du bois, reliés à des moyens (13, 14, 15) d'analyse de l'intensité du flux agencés pour en déduire une valeur d'humidité, comportant des moyens de présentation de ladite valeur.

## Description

La présente invention concerne un dispositif et un procédé de mesure de l'humidité du bois. Le bois qui est testé se présente notamment sous la forme de bûche, de copeaux, de sciure.

Les fabricants et les utilisateurs d'appareils de chauffage à bois tels que, par exemple, les cheminées à foyer ouvert ou fermé, les fourneaux et les poêles, souhaitent connaître le taux d'humidité du bois à brûler car l'efficacité de la combustion en dépend. Cela permet de mener un foyer, notamment du type fermé, dans les meilleures conditions de combustion possibles, car l'utilisateur peut agir à bon escient sur les inductions d'air et éventuellement sur d'autres variables sensibles du processus de combustion.

Il existe des analyseurs de fumée permettant de mesurer la composition des gaz de combustion et donc l'humidité, mais ils sont coûteux et l'accès aux gaz nécessite des manipulations parfois difficiles.

La présente invention propose une autre solution.

A cet effet, l'invention concerne tout d'abord un dispositif de mesure de l'humidité du bois, comprenant des circuits de sonde, agencés pour être mis en contact avec une pièce de bois à tester afin d'en recevoir un flux d'un courant fonction d'un taux d'humidité du bois, reliés à des moyens d'analyse de l'intensité du flux agencés pour en déduire une valeur d'humidité, comportant des moyens de présentation de ladite valeur, caractérisé en ce que les circuits de sonde sont agencés pour établir le contact par placage contre une surface humide de la pièce de bois.

Ainsi, il suffit de plaquer les circuits de sonde, constituant des palpeurs, sur une surface limitant un volume humide pour obtenir le résultat de mesure requis. Au besoin, pour une mesure plus précise, à coeur, ce volume peut être préalablement tranché. Comme cela apparaîtra plus loin, le flux de courant peut être un flux d'humidité ou un flux de courant électrique dont l'intensité est sensible à l'humidité.

Dans une forme de réalisation particulièrement simple, et peu coûteuse, le dispositif est de type passif et comprend une bandelette, en matériau apte à capter et à faire migrer l'humidité, comportant une zone de capture de l'humidité, agencée pour servir de dits circuits de sonde, et une zone adjacente, de dits moyens d'analyse, imprégnée d'un produit révélateur de l'humidité, destinée à la migration longitudinale de l'humidité captée.

Il est alors aisé de prévoir d'appliquer sur le bois la zone de capture pendant une durée prédéterminée pour ensuite mesurer la longueur de migration et en déduire l'humidité du bois, puisque la vitesse de migration dans la zone de migration dépend de la quantité d'eau disponible par unité de temps par extraction par contact avec le bois qui, elle-même, traduit le taux d'humidité du bois, cette disponibilité étant également fonction de la nature du bois et de son aptitude à relâcher l'humidité.

Ainsi, l'humidité pénètre dans la bandelette latéralement selon la direction de l'épaisseur de celle-ci et migre ensuite selon sa direction longitudinale, en définissant ainsi un front de migration dont la position peut être lue par une observation latérale, d'un côté ou de l'autre de la bandelette, c'est-à-dire, très approximativement, selon une direction parallèle à la direction d'entrée.

Dans une forme de réalisation, il est prévu une enveloppe d'étanchéité à l'humidité, pour protéger la bandelette au stockage, enveloppe comportant, de préférence, une échelle graduée de mesure de la migration de l'humidité.

On évite ainsi la nécessité d'un stockage en atmosphère contrôlée. Par ailleurs, on évite ainsi la nécessité de disposer d'une règle séparée, ou équivalent, portant une telle échelle, qui offre donc une meilleure précision qu'une estimation, sans repère annexe, de la longueur de migration.

En variante, l'échelle graduée de mesure de la migration de l'humidité est portée par la zone de migration.

Avantageusement, l'échelle est graduée en unités de longueur. La mesure de la longueur, qui représente un résultat intermédiaire, permet de déduire un taux d'humidité et ceci pour une ou plusieurs durées de contact du bois avec la zone de capture.

En variante, l'échelle peut être graduée en taux d'humidité pour une mesure directe de l'humidité, valable pour un contact de durée prédéterminée entre le bois et la zone de capture, chacune des échelles correspondant à une nature de bois déterminée.

La zone de capture peut être située sur une seule face de la bandelette ou être répartie sur les deux faces de la bandelette.

Lorsque l'échelle est portée par l'enveloppe, le dispositif comporte de préférence des moyens de cadrage de la bandelette par rapport à l'enveloppe, et donc par rapport à l'échelle.

L'enveloppe peut être prévue pour être fendue à un endroit prédéterminé pour permettre l'extraction d'un tronçon d'extrémité avant de la bandelette constituant la zone de capture, les moyens de cadrage comportent un lien de limitation de l'extraction, reliant la bandelette à l'enveloppe, de longueur limitée pour interdire l'extraction de la zone de migration. Le lien peut, par exemple, relier l'arrière de la bandelette au fond de l'enveloppe ou peut occuper tout autre position relative.

En variante, la zone de migration peut présenter une largeur plus grande que celle de la zone de capture pour former un épaulement de butée contre le bord d'une fente d'extraction de l'enveloppe prévue pour le passage de la zone de capture.

Avantageusement, le produit révélateur de l'humidité est le chlorure de cobalt.

Dans une autre forme de réalisation du dispositif selon l'invention, alors de type actif, les circuits de sonde comprennent deux palpeurs reliés séparément à un circuit d'injection d'un courant de mesure commandant un circuit électronique d'analyse agencé pour mesurer la puissance électrique du courant, pour en déduire une valeur de résistance électrique et y appliquer une conversion fournissant une valeur de mesure d'humidité, et agencé pour commander un circuit de fourniture d'informations, c'est-à-dire de relations homme-machine, selon ladite valeur de mesure d'humidité.

Le dispositif comporte un nombre limité d'éléments et peut ainsi être fabriqué sous une forme compacte et facile d'utilisation.

Le circuit d'injection peut être une source de tension de valeur fixe et le circuit d'analyse est alors prévu pour mesurer le courant correspondant.

En variante, le circuit d'injection est une source de courant de valeur fixe et le circuit d'analyse est prévu pour mesurer la tension correspondante entre les deux palpeurs.

Avantageusement, le circuit d'injection de courant et le circuit électronique d'analyse sont intégrés dans un même circuit intégré.

Une compacité optimale est ainsi obtenue.

Le dispositif peut comporter des moyens de sélection d'une échelle pour la dite conversion, en fonction de l'essence du bois.

Pour éviter la nécessité d'une pile, il peut être prévu un capteur photovoltaïque d'alimentation des dits circuits.

Il peut en outre être prévu une carte de support et de liaison mutuelle des circuits. La carte peut être en matériau souple.

Les palpeurs peuvent ainsi être constitués par deux zones de la carte, par exemple en bout, et ils sont ainsi agencés pour s'adapter aux reliefs de surface du bois.

L'invention concerne aussi un procédé de mesure de l'humidité du bois, dans lequel, disposant d'un dispositif de mesure de l'humidité de type passif selon l'invention,
a) l'utilisateur ouvre l'enveloppe, si tel est le cas, pour démasquer la zone de capture,
b) l'utilisateur applique la zone de capture sur une surface du bois pendant une durée déterminée, et
c) l'utilisateur détermine, de préférence avec une échelle graduée de mesure de la migration de l'humidité, la longueur de propagation de l'humidité dans la zone de migration de la bandelette par un changement de couleur, ou, plus généralement, d'état ou aspect discernable à l'oeil, du produit révélateur de l'humidité dans la direction de migration et en déduit un taux d'humidité.

La ligne de transition constituant le front de migration maximale de l'humidité fournit, par elle seule, l'information de taux d'humidité recherchée, la zone ou surface de migration déjà balayée par cette ligne n'apportant donc pas d'information supplémentaire.

Ainsi, le dispositif de l'invention permet à un utilisateur de déterminer le taux d'humidité d'une bûche de bois afin de conditionner celle-ci pour un séchage, si le taux d'humidité est jugé trop important par l'utilisateur pour une gestion efficace d'un foyer de combustion, ou bien d'agir sur le réglage des inductions d'air pour optimiser la combustion en fonction de l'humidité résiduelle et de la nature du bois utilisé.

L'invention sera mieux comprise à l'aide de la description suivante de deux formes de réalisation intéressantes de l'invention, en référence aux dessins annexés, sur lesquels :
- la figure 1 est une vue de dessus de la première forme de réalisation du dispositif, alors passif, de mesure de l'humidité du bois selon l'invention,
- la figure 2 est une vue de dessus du dispositif de la figure 1, en position fonctionnelle d'utilisation, et
- la figure 3 est un diagramme schématique illustrant la seconde forme de réalisation du dispositif selon l'invention.

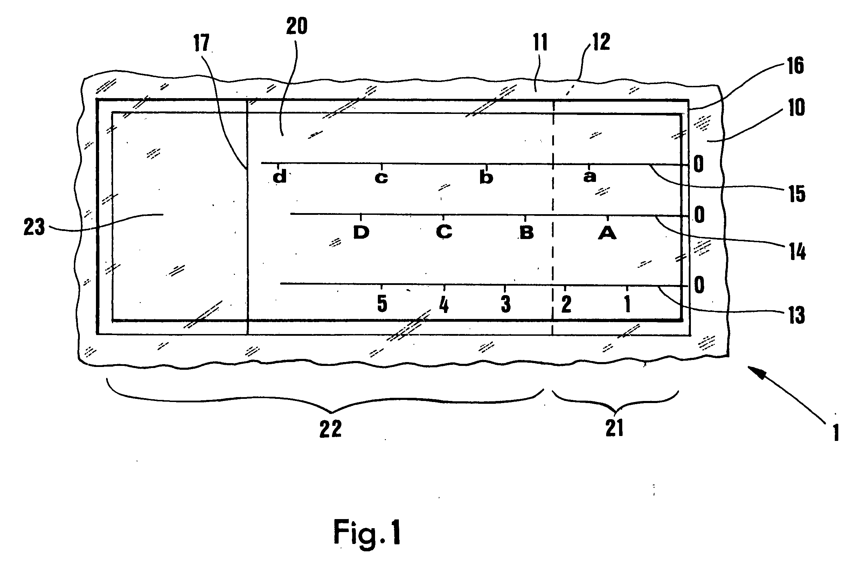

Sur la figure 1, un dispositif 1 selon la première forme de réalisation du dispositif 1 comporte une bandelette 20 détectrice d'humidité, ici rectangulaire, protégée par une enveloppe étanche à l'humidité 10.

La bandelette 20 est en matériau apte à capter l'humidité dans une zone de capture 21, ici à une extrémité du rectangle, et à la faire migrer, ou propager, dans une zone de migration 22 occupant le reste du rectangle, c'est-à-dire adjacente par rapport à la zone de capture 21. La capture de l'humidité est effectuée par mise en contact du bois à tester avec la zone de capture 21. La bandelette 20 est réalisée en un matériau présentant un pouvoir d'absorption de l'eau autorisant une migration de l'humidité, c'est-à-dire permettant une extension de la surface de la zone humide au-delà de la limite de la zone de capture 21, dans la zone périphérique 22. La bandelette 20 capte l'humidité par une surface de contact de taille déterminée, représentant une fraction de la surface totale de la bandelette 20 et l'eau ainsi absorbée migre longitudinalement, donc selon la longueur du rectangle, délimitant un front de migration.

Pour permettre la mesure de la longueur de migration, la bandelette 20 est préimprégnée d'un produit révélateur d'humidité, qui change d'aspect en présence d'humidité, c'est-à-dire au-dessus d'un seuil prédéterminé.

L'enveloppe scellée 10, ici de type étui protecteur, est formée ici de deux demi feuilles identiques 11, 12 en matériau étanche aux fluides, thermosoudées entre elles sur leur pourtour pour délimiter un volume intérieur protégé de l'humidité. Le volume intérieur ainsi protégé contient, avant utilisation, la bandelette 20 de taille très légèrement inférieure à celle des deux demi feuilles 11, 12.

Dans cet exemple, l'enveloppe 10 est en matière plastique connue de l'homme du métier, par exemple de type polyéthylène.

Pour mesurer la distance de migration, il est ici prévu une échelle 13 graduée en unités de longueur, ici des centimètres et numérotés de 0 à 5, portée dans cet exemple par l'enveloppe 10, le début de l'échelle 13 étant situé à une extrémité avant 16, de sortie de la bandelette 20, de l'enveloppe 10. Il peut également être prévu au moins une autre échelle, dans cet exemple deux échelles 14, 15, également portées par l'enveloppe 10, graduées directement en unités de taux d'humidité, ici représentées respectivement par 0, A, B, C, D et 0, a, b, c, d, dont les origines des graduations sont également situées à l'extrémité avant 16 de l'enveloppe 10. Les échelles 14, 15 sont chacune fonction de la texture du bois possible à tester, c'est-à-dire de son aptitude à propager l'humidité qu'il contient jusqu'à la zone de capture 21. C'est donc fonction de la quantité d'eau capturée par la zone de capture 21 et provenant de la masse du bois sous test. Ce débit dépend, en particulier, de la transmission de l'eau dans le réseau fibreux du bois, qui influe sur la vitesse de migration de l'humidité jusqu'à la surface de capture 21. Les échelles graduées 13, 14, 15 portées par l'enveloppe 10 peuvent, par la suite, être appliquées sur la zone de migration 22, par cadrage de l'enveloppe 10 par rapport à la bandelette 20. Le cadrage peut être réalisé par ajustement, par l'utilisateur, des positions relatives entre la bandelette 20 et l'enveloppe 10. Pour faciliter cet ajustement, il est prévu ici un marquage 17 de la limite de la zone de capture 21. Le marquage 17 permettra par la suite à l'utilisateur, faisant sortir la bandelette 20 sur une distance déterminée au niveau de l'extrémité avant 16 de l'enveloppe 10 une fois ouverte, de définir la zone de capture 21 qui sera ainsi toujours identique et reproductible. Dans un tel cas, l'enveloppe 10 est de préférence en matériau transparent.

Selon une autre forme de réalisation du dispositif, les échelles graduées 13, 14, 15 peuvent être intégrées dans la zone de migration 22 de la bandelette 20, pour une mesure directe de la longueur de migration de l'humidité. Dans ce cas, le marquage 17, qui figurait sur l'enveloppe 10, n'est plus nécessaire, l'utilisateur faisant partiellement sortir la bandelette 20 au niveau de l'extrémité avant 16 de l'enveloppe 10 une fois ouverte jusqu'à faire coïncider l'origine des échelles 13, 14, 15 avec l'extrémité avant 16 de l'enveloppe 10. En variante, il peut être prévu des moyens de cadrage (non dessinés) de la bandelette 20 par rapport à l'enveloppe 10. Les moyens de cadrage peuvent être un lien de longueur limité qui retient l'extrémité arrière 23 de la bandelette 20 dès que la zone de capture 21 a été sortie de l'enveloppe par l'extrémité 16 de l'enveloppe 10, fendue selon sa largeur.

En variante encore, la zone de capture 21 présente une largeur réduite par rapport à la zone de migration 22, c'est-à-dire que celle forme au moins un épaulement latéral qui vient buter sur la zone de paroi d'enveloppe 10 prolongeant l'ouverture dont l'extension correspond à la largeur de la zone de capture 21. L'enveloppe 10 sert de glissière pour maintenir l'orientation globale de la bandelette 20.

En variante, l'enveloppe 10 peut être formée d'une couche transparente de type vernis ou équivalent couvrant la zone de migration 22 et par un cache amovible couvrant la zone de capture 21.

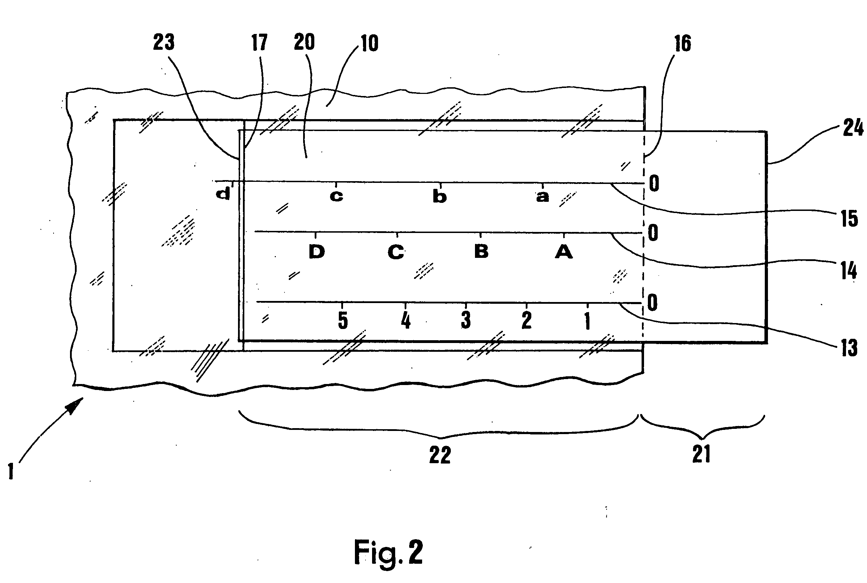

La figure 2 représente le dispositif 1 en position fonctionnelle de mesure de l'humidité dans laquelle la bandelette 20 est partiellement sortie de l'enveloppe 10 après que celle-ci a été ouverte au niveau de l'extrémité avant 16, de sortie de la bandelette 20, de l'enveloppe 10 jusqu'à faire coïncider l'extrémité arrière 23 de la bandelette 20 avec le marquage 17 porté sur l'enveloppe 10. Dans un souci de clarté, le marquage 17 et l'extrémité arrière 23 de la bandelette 20 ont été distingués l'un de l'autre. Cette configuration permet de définir la zone adjacente de migration 22 de l'humidité, délimitée par l'extrémité arrière 23 de la bandelette 20 et l'extrémité avant 16 de l'enveloppe 10, ici représentée en pointillés, et la zone de capture 21 de l'humidité. La zone de capture 21 est délimitée par l'extrémité avant 24 de la bandelette 20 et par l'extrémité avant 16 de l'enveloppe 10.

Un autre objet de l'invention concerne un procédé de mesure de l'humidité du bois, dans cet exemple une bûche de bois, susceptible d'être mis en oeuvre en utilisant le dispositif de l'invention. Dans ce qui suit, le taux d'humidité d'une bûche de bois, pouvant être exprimé comme le pourcentage d'humidité relative en poids, s'entend comme étant le taux moyen d'humidité de la bûche car celui-ci pourra être différent si la mesure est effectuée au niveau de la périphérie ou au coeur même du bois.

Préalablement à la mesure de l'humidité d'une bûche de bois à proprement parler, l'utilisateur se procure une bûche de bois ou plusieurs bûches d'essences différentes déterminées présentant un diamètre moyen à titre d'exemple d'environ 10 à 15 cm représentatives de son stock qu'il se propose de brûler dans un laps de temps et des conditions de stockage dont les caractéristiques, par exemple l'humidité ambiante, seraient alors susceptibles d'être modifiées. L'utilisateur fend ensuite cette bûche de bois en deux parties, ou morceaux, en son milieu et dans le sens de la longueur dans un local sec.

L'utilisateur s'étant muni du dispositif 1, il en ouvre l'extrémité avant 16 de l'enveloppe 10, par exemple avec un objet coupant de type paire de ciseaux. L'ouverture de l'extrémité de l'enveloppe 10 permet à l'utilisateur de faire sortir partiellement la bandelette 20 de son étui protecteur 10 sur une distance déterminée, définissant la zone de capture 21 de l'humidité, tandis que la zone de migration 22 reste protégée de l'humidité ambiante par l'enveloppe 10.

L'utilisateur applique ensuite la zone de capture 21 de l'humidité entre deux zones opposées des deux morceaux fendus de la bûche de bois. Pour assurer le contact optimal entre les deux morceaux de la bûche et la zone de capture 21 de la bandelette 20, l'utilisateur doit s'assurer de remettre en coïncidence les deux morceaux de la bûche tels qu'ils étaient avant que la bûche ne soit fendue. Le contact et le maintien de la zone de capture 21 avec les deux zones des morceaux de la bûche sont assurés de façon manuelle ou par l'intermédiaire d'un quelconque moyen de mise en contact et de maintien.

La mise en contact de la zone de capture 21 de la bandelette 20 avec les deux parties de la bûche provoque une humidification de la zone de capture 21 et, par conséquent, la migration ou propagation longitudinale de l'humidité dans la zone de migration 22. Le produit sensible à l'humidité, ici le chlorure de cobalt, imprégnant la bandelette 20, passe de la couleur bleue témoin, c'est-à-dire en l'absence d'humidité, à la couleur rose lorsqu'il est en contact avec le flux humide au fur et à mesure de la propagation de ce dernier.

Après une période d'application de 5 minutes, l'utilisateur estime donc la distance parcourue par l'humidité dans la zone de capture 22 grâce au changement de couleur du produit révélateur d'humidité et le front de migration est en outre repéré par au moins l'une des échelles 13, 14, 15 ici portées par l'enveloppe 10. Si l'essence de bois correspond à celle pour laquelle l'une des échelles graduées 14, 15 de mesure du taux d'humidité est prévue, l'utilisateur en déduit directement le pourcentage relatif d'humidité en poids. Par contre, dans le cas contraire, c'est-à-dire dans le cas ou l'essence de bois de la bûche ne correspond pas à celle prévue pour une mesure directe, il sera aisé de déduire de la position du front de migration sur l'échelle 13, un pourcentage relatif d'humidité à l'aide d'une table de correspondance prévue à cet effet, et fournie avec le dispositif de l'invention, la table indiquant, pour une distance de migration donnée et éventuellement pour une pluralité de temps de contact avec la bûche de bois sous test, le pourcentage relatif d'humidité en poids d'une pluralité d'essences de bois considérée.

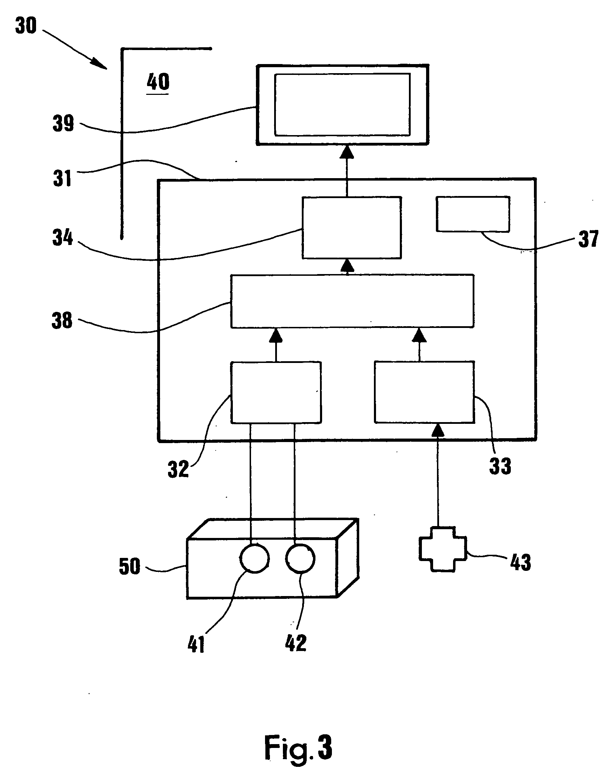

La figure 3 est un diagramme schématique du dispositif selon la seconde forme de réalisation de l'invention.

Un dispositif 30 selon seconde forme de réalisation comporte, dans cet exemple, une carte 40 de support et de liaison électrique des divers circuits représentés. Le dispositif 30 comporte des circuits de sonde comprenant deux palpeurs 41, 42 reliés séparément à un circuit 32 d'injection d'un courant de mesure commandant un circuit électronique d'analyse 38 agencé pour mesurer la puissance électrique du courant, pour en déduire une valeur de résistance électrique et y appliquer une conversion fournissant une valeur de mesure d'humidité, et agencé pour commander un afficheur 39 selon la valeur de mesure d'humidité. La référence 50 désigne une pièce de bois sur une surface de laquelle sont appliqués les deux palpeurs 41, 42, qui peuvent être portés par un même film support.

Le circuit d'injection de courant 32 est ici intégré dans un circuit intégré 31 dont le circuit électronique d'analyse 38 constitue une unité centrale.

Le circuit d'injection 32 est une source de tension de valeur fixe et le circuit d'analyse 38 est prévu pour mesurer le courant correspondant, continu ou alternatif.

En variante, le circuit d'injection 32 est une source de courant de valeur fixe et le circuit d'analyse 38 est prévu pour mesurer la tension correspondante, continue ou alternative, entre les deux palpeurs 41, 42.

Dans cet exemple, il est prévu comportant des circuits de sélection d'une échelle pour la dite conversion, en fonction de l'essence du bois, à savoir une touche ou un clavier 43 commandant un circuit d'interface d'entrée 33 du circuit intégré 31, relié au circuit d'analyse 38.

L'alimentation électrique des divers circuits est ici assurée par un capteur photovoltaïque 37, associé à un condensateur réservoir non dessiné.

Le circuit d'analyse 38 commande un circuit d'interface de sortie prévu pour commander de façon appropriée l'afficheur 39.

En variante, les circuits d'interface de sortie 34 sont un synthétiseur vocal commandant un petit haut-parleur.

Le fonctionnement du dispositif 30 va maintenant être exposé plus en détails.

Les deux palpeurs 41, 42 étant appliqués sous pression entre la surface de la pièce de bois 50, une boucle de courant est ainsi établie, la résistance présentée étant fonction de l'humidité du bois, et en particulier du contact bois / palpeurs 41, 42.

Le circuit d'analyse 38 mesure alors la variable de boucle, c'est-à-dire le flux de électronique, sortant et revenant après traversée du bois, et la convertit en une valeur d'humidité, exprimée sous la forme d'un taux d'humidité. La pente de conversion dépend d'une commande préalablement reçue du clavier 43. Comme évoqué, le clavier 43, éventuellement intégré dans l'afficheur 39, alors de type à écran sensitif, peut se limiter à une seule touche servant à incrémenter, par des commandes impulsionnelles pas à pas, la pente ci-dessus puis à en commander le décrément si la valeur maximale a été atteinte.

La mesure s'affiche par exemple sous la forme numérique d'un nombre ou encore d'un nombre variable de barres successives dans une échelle de barres ou uniquement de la barre la plus "haute", de limite.

Les palpeurs 41, 42 sont de préférence disposés en bout de la carte 40, pour que le circuit intégré ne constitue pas une gêne lors de leur pose sur le bois.

En variante, le dispositif 30 se présente sous la forme d'une pastille comportant les circuits ci-dessus, les palpeurs 41, 42 étant disposés sur une face de la pastille.

## Revendications

1. Dispositif (1, 30) de mesure de l'humidité du bois, comprenant des circuits de sonde (21, 32, 41, 42), agencés pour être mis en contact avec une pièce de bois (50) à tester afin d'en recevoir un flux d'un courant fonction d'un taux d'humidité du bois, reliés à des moyens (13, 14, 15, 39, 22, 31) d'analyse de l'intensité du flux agencés pour en déduire une valeur d'humidité, comportant des moyens de présentation de ladite valeur, **caractérisé en ce que** les circuits de sonde (21, 32, 41, 42) sont agencés pour établir le contact par placage contre une surface humide de la pièce de bois (50).

2. Dispositif selon la revendication 1, comprenant une bandelette (20), en matériau apte à capter et à faire migrer l'humidité, comportant une zone (21) de capture de l'humidité, agencée pour servir de dits circuits de sonde, et une zone adjacente (22), de dits moyens d'analyse, imprégnée d'un produit révélateur de l'humidité, destinée à la migration longitudinale de l'humidité captée.

3. Dispositif selon la revendication 2, dans lequel une enveloppe (10) d'étanchéité à l'humidité, pour protéger la bandelette (20) au stockage, comporte une échelle graduée (13, 14, 15) de mesure de la migration de l'humidité.

4. Dispositif selon la revendication 2, dans lequel une échelle graduée (13, 14, 15) de mesure de la migration de l'humidité est portée par la zone de migration (22).

5. Dispositif selon l'une des revendications 3 et 4, dans lequel l'échelle (13) est graduée en unités de longueur.

6. Dispositif selon l'une des revendications 3 et 4, dans lequel l'échelle (14, 15) est graduée en taux d'humidité pour une mesure directe de l'humidité, valable pour un contact de durée prédéterminée entre le bois et la zone de capture (21), chacune des échelles (14, 15) correspondant à une nature de bois déterminée.

7. Dispositif selon l'une des revendications 2 à 6, dans lequel la zone de capture (21) est située sur une seule face de la bandelette (20).

8. Dispositif selon l'une des revendications 2 à 6, dans lequel la zone de capture (21) est répartie sur les deux faces de la bandelette (20).

9. Dispositif selon l'une des revendications 3 à 8, dans lequel, l'échelle (13, 14, 15) étant portée par l'enveloppe (10), il est prévu des moyens de cadrage de la bandelette (20) par rapport à l'enveloppe (10).

10. Dispositif selon la revendication 9, dans lequel, l'enveloppe (10) étant prévue pour être fendue à un endroit prédéterminé pour permettre l'extraction d'un tronçon d'extrémité avant (24) de la bandelette (20) portant la zone de capture (21), les moyens de cadrage comportent un lien de limitation de l'extraction, reliant la bandelette (20) à l'enveloppe (10), de longueur limitée pour interdire l'extraction de la zone de migration (22).

11. Dispositif selon la revendication 9, dans lequel la zone de migration (22) présente une largeur plus grande que celle de la zone de capture (21) pour former un épaulement de butée contre le bord d'une fente d'extraction de l'enveloppe (10) prévue pour le passage de la zone de capture (21).

12. Dispositif selon l'une quelconque des revendications 2 à 11, **caractérisé en ce que** le produit révélateur de l'humidité est le chlorure de cobalt.

13. Dispositif selon la revendication 1, dans lequel les circuits de sonde comprennent deux palpeurs (41, 42) reliés séparément à un circuit (32) d'injection d'un courant de mesure commandant un circuit électronique d'analyse (38) agencé pour mesurer la puissance électrique du courant, pour en déduire une valeur de résistance électrique et y appliquer une conversion fournissant une valeur de mesure d'humidité, et agencé pour commander un circuit de fourniture d'informations (39) selon ladite valeur de mesure d'humidité.

14. Dispositif selon la revendication 13, dans lequel le circuit d'injection (32) est une source de tension de valeur fixe et le circuit d'analyse (38) est prévu pour mesurer le courant correspondant.

15. Dispositif selon la revendication 13, dans lequel le circuit d'injection (32) est une source de courant de valeur fixe et le circuit d'analyse (38) est prévu pour mesurer la tension correspondante entre les deux palpeurs (41, 42).

16. Dispositif selon l'une des revendications 13 à 15, dans lequel le circuit d'injection de courant (32) et le circuit électronique d'analyse (38) sont intégrés dans un même circuit intégré.

17. Dispositif selon l'une des revendications 13 à 16, comportant des moyens (33, 43) de sélection d'une échelle pour la dite conversion, en fonction de l'essence du bois.

18. Dispositif selon l'une des revendications 13 à 17, comportant un capteur photovoltaïque d'alimentation des dits circuits.

19. Dispositif selon l'une des revendications 13 à 18, comportant une carte de support et de liaison mutuelle des circuits.

20. Dispositif selon la revendication 19, dans lequel la carte est en matériau souple.

21. Procédé de mesure de l'humidité du bois, dans lequel, disposant d'un dispositif (1) de mesure de l'humidité selon l'une quelconque des revendications 2 à 12,
a) l'utilisateur ouvre l'enveloppe (10), si tel est le cas, pour démasquer la zone de capture (21),
b) l'utilisateur applique une surface du bois sur la zone de capture (21) pendant une durée déterminée, et
c) l'utilisateur détermine, d'après une échelle graduée de mesure de la migration de l'humidité, la longueur de propagation de l'humidité dans la zone de migration (22) de la bandelette (20) par un changement d'aspect du produit révélateur de l'humidité dans la direction de migration et en déduit un taux d'humidité du bois.
